(19) Europäisches Patentamt
European Patent Office
Office européen des brevets

(11) **EP 3 675 811 B1**

(12) **FASCICULE DE BREVET EUROPEEN**

(45) Date de publication et mention de la délivrance du brevet:
**02.03.2022   Bulletin 2022/09**

(21) Numéro de dépôt: **18762295.6**

(22) Date de dépôt: **30.08.2018**

(51) Classification Internationale des Brevets (IPC):
**A61K 8/65** (2006.01)       **A61K 8/44** (2006.01)
**A61K 8/64** (2006.01)       **A61Q 5/00** (2006.01)
**A61Q 19/00** (2006.01)       **A23L 33/18** (2016.01)
**A23L 33/175** (2016.01)

(52) Classification Coopérative des Brevets (CPC):
**A61K 8/65; A23L 33/175; A23L 33/18; A61K 8/44; A61K 8/447; A61K 8/64; A61Q 5/00; A61Q 19/00;**
A61K 2800/92

(86) Numéro de dépôt international:
**PCT/EP2018/073406**

(87) Numéro de publication internationale:
**WO 2019/043128 (07.03.2019 Gazette 2019/10)**

(54) **HYDROLYSAT DE KÉRATINE POUR UTILISATION COSMÉTIQUE PAR VOIE ORALE**

KERATINHYDROLYSAT ZUR ORALEN KOSMETISCHEN VERWENDUNG

KERATIN HYDROLYSATE FOR ORAL COSMETIC USE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.09.2017   FR 1770921**

(43) Date de publication de la demande:
**08.07.2020   Bulletin 2020/28**

(73) Titulaire: **Bretagne Chimie Fine**
**56140 Pleucadeuc (FR)**

(72) Inventeur: **SERGHERAERT, Renaud**
**56870 Baden (FR)**

(74) Mandataire: **Opilex**
**32, rue Victor Lagrange**
**69007 Lyon (FR)**

(56) Documents cités:
**WO-A1-2007/083402       DD-A1- 244 760**

**FR-A1- 2 704 394       US-A- 1 608 686**
**US-A- 4 245 117**

- BARBA C ET AL: "Effect of wool keratin proteins and peptides on hair water sorption kinetics", JOURNAL OF THERMAL ANALYSIS AND CALORIMETRY, KLUWER ACADEMIC PUBLISHERS, DORDRECHT, NL, vol. 102, no. 1, 30 janvier 2010 (2010-01-30), pages 43-48, XP019825147, ISSN: 1572-8943
- EMIL ABDERHALDEN ET AL: "Hydrolyse des Keratins aus Horn und aus Wolle", HOPPE-SEYLER'S ZEITSCHRIFT FUER PHYSIOLOGISCHE CHEMIE., vol. 52, no. 3-4, 1 janvier 1907 (1907-01-01), pages 348-367, XP055471234, DE ISSN: 0018-4888, DOI: 10.1515/bchm2.1907.52.3-4.348
- "New hand, nail care ingredient aids against drying effect of detergents", FOCUS ON SURFACTANTS, ELSEVIER, AMSTERDAM, NL, vol. 2005, no. 10, 1 octobre 2005 (2005-10-01), page 4, XP027740473, ISSN: 1351-4210 [extrait le 2005-10-01]

**Description**

La présente invention se rapporte au domaine des hydrolysats de kératine et plus particulièrement à l'utilisation cosmétique, par voie orale, d'un hydrolysat de kératine comme agent pour améliorer la qualité, l'aspect des ongles et/ou des cheveux. Elle concerne également un complément alimentaire comprenant cet hydrolysat de kératine.

Art antérieur

Les cheveux et les ongles font partie de la famille des phanères qui sont des organes qui protègent la peau du milieu extérieur. L'aspect des phanères, et notamment des cheveux, compte parmi les principaux critères communément liés à la beauté.

Afin d'obtenir et de conserver une chevelure de qualité, de nombreuses compositions destinées à être appliquées sur les cheveux telles que des shampooings, après-shampooings, baumes, huiles ont été développées.

Il existe aussi des compléments alimentaires à base de vitamines(s) B et/ou d'acides aminés soufrés dédiés à améliorer la qualité des phanères, notamment des compléments alimentaires à base de kératine sont proposés à la vente. Ces compléments alimentaires à base de kératine sont décrits comme comprenant les 17 acides aminés essentiels principaux dont la cystéine en une quantité significative. Le document WO2007/88402 décrit des compléments alimentaires à base de poudre de coquille d'oeuf, d'un hydrolysat de kératine et d'un extrait de ginko.

Il demeure toujours un besoin de disposer de produits performants pour améliorer la qualité des phanères.

La présente invention vise à répondre à ce besoin.

Ainsi les inventeurs ont mis en œuvre un hydrolysat de kératine dont la composition particulière permet une amélioration avantageuse de l'aspect des phanères notamment la beauté, la qualité, la résistance des cheveux et des ongles, et plus particulièrement une amélioration de l'éclat, l'ancrage, le volume, la densité et la brillance des cheveux.

D'autres aspects, avantages, propriétés de la présente invention sont présentés dans la description et les exemples qui suivent.

Résumé de l'invention

La présente invention a donc pour objet un hydrolysat de kératine présentant les caractéristiques suivantes :

ledit hydrolysat comprend au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton,

ledit hydrolysat comprend de la L-cystine en une teneur allant de 4 à 6% en poids par rapport au poids total de l'hydrolysat,

ledit hydrolysat comprend de la cystéine en une teneur inférieure ou égale à 0,1% en poids par rapport au poids total de l'hydrolysat,

ledit hydrolysat comprend de la tyrosine en une teneur inférieure ou égale à 0,6% en poids par rapport au poids total de l'hydrolysat.

Les inventeurs ont montré que, de manière surprenante, cet hydrolysat permet de conférer des propriétés particulièrement avantageuses aux phanères. L'hydrolysat de kératine selon l'invention permet l'obtention de cheveux plus brillants, mieux ancrés à la papille pilaire, la chevelure apparaît plus volumineuse.

Les ongles apparaissent également plus brillants.

La présente invention vise encore un procédé de préparation de l'hydrolysat de kératine selon l'invention à partir d'une matière première kératinique de volaille, ledit procédé comprenant au moins les étapes suivantes, dans cet ordre :

- soumettre la matière première à au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton ;

- extraire la tyrosine dudit hydrolysat ;

- éventuellement sécher.

Selon un troisième objet, l'invention vise l'utilisation cosmétique, par voie orale, de l'hydrolysat de kératine selon l'invention comme agent pour améliorer la qualité et/ou l'aspect des ongles et/ou des cheveux, notamment pour améliorer l'ancrage, la brillance et/ou le volume et/ou la densité des cheveux.

Un quatrième objet de l'invention vise un complément alimentaire, notamment dédié à améliorer l'aspect des ongles et/ou des cheveux, comprenant un hydrolysat de kératine selon l'invention ou obtenu suivant le procédé de préparation selon l'invention, le complément alimentaire comprend de 40 à 60% en poids d'hydrolysat de kératine, par rapport au poids total dudit complément alimentaire.

Selon un cinquième objet, l'invention vise un procédé de traitement cosmétique pour améliorer la qualité et/ou l'aspect des ongles et/ou des cheveux, notamment pour améliorer la qualité des cheveux, comprenant l'administration, par voie orale, à un individu, d'un hydrolysat selon la présente invention ou d'un complément alimentaire selon la présente invention.

D'autres objets, aspects, avantages, propriétés de la présente invention sont présentés dans la description et les exemples qui suivent.

Description détaillée

**Hydrolysat**

Comme déjà mentionné, une des caractéristiques principales de l'hydrolysat de kératine selon la présente invention est qu'il présente des teneurs très basses en deux acides aminés : la tyrosine et la cystéine.

La tyrosine est ainsi présente dans l'hydrolysat de kératine selon la présente invention en une teneur inférieure ou égale à 0,6% en poids, de préférence inférieure ou égale à 0,5% en poids et de manière préférée inférieure ou égale à 0,4% en poids par rapport au poids total de l'hydrolysat. Cette faible teneur en tyrosine est la conséquence de la mise en œuvre d'une étape d'extraction de la tyrosine dans le procédé de préparation de l'hydrolysat.

Avantageusement, l'hydrolysat ne contient pas de tyrosine, les seules traces de cet acide aminé étant dues aux limites des conditions opératoires, du matériel mis en œuvre lors de l'étape d'extraction.

La cystéine est présente dans l'hydrolysat de kératine selon la présente invention en une teneur inférieure ou égale à 0,1%, de préférence inférieure ou égale 0,05% en poids par rapport au poids total de l'hydrolysat, de manière encore préférée, l'hydrolysat ne contient pas de cystéine.

Par contre, l'hydrolysat selon la présente invention comprend de la L-cystine, qui est un dimère de la cystéine, en une teneur significative. En effet, la teneur en L-cystine va de 4 à 6% en poids, de préférence de 4,5% à 5,5% en poids par rapport au poids total de l'hydrolysat.

L'hydrolysat selon la présente invention comprend au moins 88%, de préférence au moins 93% et de manière encore préférée au moins 95% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton.

En effet, l'hydrolysat comprend essentiellement des acides aminés : des acides aminés libres (au moins 88% en poids par rapport au poids total des acides aminés) et des acides aminés liés pour former des peptides, l'hydrolysat comprenant en outre de la matière minérale et de l'eau.

Avantageusement, la teneur en acides aminés totaux (libres et liés) de l'hydrolysat va de 80% à 90% en poids par rapport au poids total de l'hydrolysat.

Selon un premier mode préféré, l'hydrolysat de kératine selon l'invention présente la composition suivante en acides aminés totaux : une teneur en glycine allant de 7 à 10 % en poids ; une teneur en alanine allant de 4 à 6 % en poids ; une teneur en valine allant de 6 à 10 % en poids ; une teneur en proline allant de 9 à 14 % en poids.

Selon un deuxième mode préféré, l'hydrolysat de kératine selon l'invention comprend la composition suivante en acides aminés totaux : une teneur en acide aspartique allant de 6 à 9 % en poids, de préférence 7,6 % en poids ; une teneur en thréonine allant de 4 à 6 % en poids, de préférence de 5,1 % en poids ; une teneur en sérine allant de 10 à 15 % en poids, de préférence de 12,4 % en poids ; une teneur en acide glutamique allant de 9 à 14 % en poids, de préférence de 11,4 % en poids ; une teneur en glycine allant de 7 à 10 % en poids, de préférence de 8,6 % en poids ; une teneur en alanine allant de 4 à 6 % en poids, de préférence de 5,0 % en poids ; une teneur en valine allant de 6 à 10 % en poids, de préférence 8,2 % en poids ; une teneur en méthionine allant de 0,1 à 0,4 % en poids, de préférence de 0,2 % en poids ; une teneur en isoleucine allant de 4 à 6 % en poids, de préférence de 4,6 % en poids ; une teneur en leucine allant de 6 à 9 % en poids, de préférence 7,7 % en poids ; une teneur en tyrosine allant de 0,1 à 0,5 % en poids, de préférence 0,3 % en poids; une teneur en phénylalanine allant de 2 à 3 % en poids, de préférence 2,5 % en poids ; une teneur en lysine allant de 1 à 3 % en poids, de préférence 2,0 % en poids, une teneur en histidine allant de 0,4 à 1 % en poids, de préférence 0,7 % en poids ; une teneur en arginine allant de 5 à 8 % en poids, de préférence 6,4 % en poids ; une teneur en proline allant de 9 à 14 % en poids, de préférence 11,8 % en poids, une teneur en tryptophane inférieure à 0.1 % ,de préférence 0 % en poids et une teneur en L-Cystine allant de 4 à 6 % en poids, de préférence 5,5 % en poids.

Selon un troisième mode préféré, l'hydrolysat de kératine selon l'invention comprend la composition suivante en acides aminés totaux : une teneur en acide aspartique allant de 6 à 9 % en poids ; une teneur en thréonine allant de 4 à 6 % en poids ; une teneur en sérine allant de 10 à 15 % en poids ; une teneur en acide glutamique allant de 9 à 14 % en poids ; une teneur en glycine allant de 7 à 10 % en poids ; une teneur en alanine allant de 4 à 6 % en poids ; une teneur en valine allant de 6 à 10 % en poids ; une teneur en méthionine de 0, 2 % en poids ; une teneur en isoleucine allant de 4 à 6 % en poids ; une teneur en leucine allant de 6 à 9 % en poids ; une teneur en tyrosine allant de 0,1 à 0,5 % en poids ; une teneur en phénylalanine allant de 2 à 3 % en poids ; une teneur en lysine allant de 1 à 3 % en poids, une teneur en histidine allant de 0,4 à 1 % en poids ; une teneur en arginine allant de 5 à 8 % en poids ; une teneur en proline allant de 9 à 14 % en poids et une teneur en L-Cystine allant de 4 à 6 % en poids.

Avantageusement, le taux de matière minérale dudit hydrolysat est inférieur ou égal à 9 % en poids, de préférence inférieure à 8% en poids par rapport au poids total de l'hydrolysat.

Le taux de matière minérale est déterminé après calcination de l'hydrolysat à 550°C pendant 4 heures.

Les acides aminés sont dosés selon une méthode adaptée du règlement CE 152/2009.

Selon cette méthode, pour la détermination des quantités d'acides aminés totaux une hydrolyse au moyen d'un acide est préalablement effectuée.

Pour la détermination des quantités d'acides aminés libres et totaux, les acides aminés sont séparés par chromatographie (CLHP ou « HPLC » en langue anglaise) avec colonne échangeuse d'ions et dosés par réaction avec la ninhydrine et détection photométrique généralement à 570 nm.

Sans vouloir être lié à une quelconque théorie, il semble qu'effectuer une hydrolyse chimique poussée permettant de conduire à ces caractéristiques, notamment une masse moléculaire des peptides présents inférieure ou égale à 800 Dalton, confère des propriétés particulières à l'hydrolysat.

Un autre avantage de l'hydrolysat selon la présente invention est qu'il très digestible, en outre, il est reconnu être de qualité alimentaire. L'hydrolysat selon l'invention présente une digestibilité vraie des acides aminés d'au moins 90%. Cette valeur est très proche du maximum possible (100%)

La digestibilité est mesurée *in vivo* selon la méthode décrite par Cozannet P., Primot Y., Gady C., Métayer J.P., Lessire M., Skiba F., Noblet J. dans « Standardised amino acid digestibility of wheat distillers' dried grains with solubles in force-fed cockerels ». British Poultry Science, Feb. 2011 ; 52(1):72-81.

Plus particulièrement l'hydrolysat de kératine selon la présente invention comprend de la L-Cystine et 16 acides aminés répartis conformément au profil d'acides aminés totaux présenté dans le tableau 1 : dans la première colonne sont présentées les gammes préférées en chacun des constituants, et dans la deuxième colonne sont présentées les teneurs préférées pour chacun des constituants.

Tableau 1 :

| Acide aminé/ peptide | Gamme de teneur dans l'hydrolysat (% poids) | Teneur préférée dans l'hydrolysat (% poids) | Acide aminé/ peptide | Gamme de teneur dans l'hydrolysat (% poids) | Teneur préférée dans l'hydrolysat (% poids) |
|---|---|---|---|---|---|
| Asp | 6-9 | 7,6 | Ile | 4-6 | 4,6 |
| Thr | 4-6 | 5,1 | Leu | 6-9 | 7,7 |
| Ser | 10 - 15 | 12,4 | Tyr | 0,1 - 0,5 | 0,3 |
| Glu | 9 - 14 | 11,4 | Phe | 2-3 | 2,5 |
| Gly | 7 - 10 | 8,6 | Lys | 1-3 | 2,0 |
| Ala | 4 - 6 | 5,0 | His | 0,4 - 1 | 0,7 |
| Val | 6 - 10 | 8,2 | Arg | 5 -8 | 6,4 |
| Met | 0,1 - 0,4 | 0,2 | Pro | 9-14 | 11,8 |
| L-Cystine | 4-6 | 5,5 | **total** | | **100** |

Contrairement à certains hydrolysats de kératine, l'hydrolysat selon la présente invention ne contient pas de sulfoxycystéine.

**Procédé de préparation de l'hydrolysat**

Comme déjà mentionné, la présente invention concerne aussi un procédé de préparation de l'hydrolysat de kératine à partir de matières kératiniques naturelles, notamment de volaille, avantageusement à partir de plumes de volaille.

Cette matière kératinique naturelle comprend majoritairement des polypeptides de haut poids moléculaires et à la structure très ramifiée la rendant peu accessible aux enzymes et qui font que ladite matière kératinique naturelle n'est pas digestible.

Avantageusement ledit hydrolysat est un hydrolysat de matière première kératinique de volaille.

A titre de volaille, on peut citer les poules, poulets, dindes, canards...

En particulier, l'hydrolysat selon la présente invention n'est pas obtenu à partir de kératine humaine tels que les cheveux.

Le procédé de préparation de l'hydrolysat de kératine selon l'invention met en œuvre au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton.

Le pourcentage de peptides dans l'hydrolysat selon l'invention va généralement de 5 à 12 % en poids par rapport au poids total de l'hydrolysat.

En effet l'hydrolyse étant partielle, le pourcentage de peptides dans l'hydrolysat n'est pas nul, de préférence il est d'au moins 5 % en poids.

L'hydrolyse chimique de la matière première est réalisée au moyen d'un acide de préférence un acide fort choisi parmi les acides chlorhydrique, phosphorique et sulfurique, de préférence l'acide chlorhydrique.

L'hydrolyse chimique est généralement effectuée pendant une durée allant de 1 heure à 8 heures, de préférence allant de 6 à 7 heures à une température allant de 110 à 115°C.

Selon une variante particulière, l'hydrolyse chimique est effectuée en deux étapes :

- une première hydrolyse chimique réalisée à une température allant de 60 à 80°C pendant une période allant de 4 à 5 heures puis

- une deuxième hydrolyse chimique réalisée à une température allant de 100 à 115°C pendant une période allant de 5 à 7 heures,

les deux hydrolyses pouvant être réalisées sans étape de pause intermédiaire ou en effectuant une étape de pause intermédiaire comprise entre 1 heure et 7 jours.

Plus précisément la première hydrolyse chimique est réalisée à 72°C pendant 4,5 heures et la deuxième hydrolyse chimique est réalisée à 107°C pendant 6 heures, une pause intermédiaire de 24 à 80 heures étant effectuées entre les deux hydrolyses chimiques.

L'étape d'extraction de la tyrosine est réalisée au moyen d'une base de préférence choisie parmi l'hydroxyde de sodium, l'hydroxyde de potassium de préférence l'hydroxyde de sodium.

L'étape d'extraction de la tyrosine est une étape classique dont la mise en œuvre relève des compétences de l'homme du métier.

Les étapes d'hydrolyse chimique et d'extraction de la tyrosine peuvent être suivies d'étapes optionnelles de purification de l'hydrolysat obtenu.

Les étapes d'hydrolyse chimique et d'extraction de la tyrosine peuvent être suivies d'étapes optionnelles de concentration de l'hydrolysat obtenu.

Les étapes d'hydrolyse chimique et d'extraction de la tyrosine peuvent être suivies d'étapes optionnelles de séchage par exemple par atomisation.

Les étapes de purification, de concentration et de séchage sont des étapes classiques dont la mise en œuvre relève des compétences de l'homme du métier.

Avantageusement, l'hydrolysat de kératine obtenu est séché par atomisation (« spray-drying » en langue anglaise) afin d'obtenir l'hydrolysat sous forme solide.

L'hydrolysat de kératine obtenu est soluble dans l'eau, en effet 1 g d'hydrolysat de kératine selon l'invention est soluble dans 5 ml d'eau.

**Utilisations**

Comme déjà mentionné, la présente invention vise l'utilisation cosmétique, par voie orale, de l'hydrolysat de kératine selon l'invention ou obtenu suivant le procédé de préparation selon l'invention comme agent pour améliorer la qualité et/ou l'aspect des ongles et/ou des cheveux, notamment pour améliorer l'ancrage, la brillance et/ou le volume et/ou la densité des cheveux.

**Complément alimentaire**

Le complément alimentaire selon la présente invention est une composition cosmétique destinée à une administration par voie orale, il peut également être qualifié de « composition nutraceutique ». Ledit complément alimentaire n'appartient pas domaine thérapeutique.

Le complément alimentaire selon la présente invention comprend au moins 28% en poids d'acides aminés libres, avantageusement entre 28% et 47,5% en poids d'acides aminés libres par rapport au poids total dudit complément alimentaire.

Avantageusement, le complément alimentaire selon la présente invention ne comprend pas d'autres acides aminés libres que ceux contenus dans l'hydrolysat à l'exception de la L-cystine qui peut être ajoutée sous forme de L-cystine additionnelle.

Le complément alimentaire selon la présente invention comprend généralement une quantité en poids de L-cystine additionnelle égale à la quantité en poids de l'hydrolysat.

De manière préférée le complément alimentaire comprend un hydrolysat de kératine selon l'invention en une teneur allant de 0,001 à 2 g, de préférence de 0,5 à 1,5 g et de manière préférée environ 0,8 à 1,2 g.

Outre l'hydrolysat de kératine selon la présente invention, le complément alimentaire selon la présente invention comprend également au moins un composant choisi dans le groupe formé par le zinc ou l'un de ses sels, le cuivre ou l'un de ses sels, les vitamines B, notamment B3, B5, B6 et/ou B8, et les mélanges de ces composants.

A titre de vitamines B utilisables dans le complément alimentaire selon la présente invention, on peut citer les vitamines B3, B5, B6 et B8 ainsi que leurs dérivés, et les mélanges de vitamines et dérivés.

De préférence, le complément alimentaire selon la présente invention comprend du zinc ou l'un de ses sels, du cuivre ou l'un de ses sels et des vitamines B3, B5, B6 et B8.

La teneur en vitamine B3 du complément alimentaire selon la présente invention va de 1 à 20 mg, de préférence de 5 à 15 mg.

La vitamine B3 (ou vitamine PP) peut être présente dans le complément alimentaire sous la forme d'un ou plusieurs composés tels que le nicotinamide (ou niacinamide), l'acide nicotinique (ou niacine), l'alcool nicotinylique, ainsi que leurs sels et dérivés.

A titre de dérivés on peut citer les esters de l'acide nicotinique tels que, par exemple, le nicotinate de tocophéryle, les acides aminés nicotiniques, les esters d'acides carboxyliques et d'alcool nicotinylique, le N-oxyde d'acide nicotinique et le N-oxydeniacinamide.

A titre de sels, on entend tout sel qui présente une innocuité adéquate.

De préférence, le nicotinamide est utilisé dans le complément alimentaire selon l'invention.

Le complément alimentaire selon la présente invention permet une administration journalière de vitamine B3 allant de 0,00001 g à 1g, de préférence de 0,0001 g à 0,5 g et de manière préférée de 0,0005 g à 0,1 g.

Avantageusement le complément alimentaire est formulé de façon à permettre une administration journalière en vitamine B3 correspondant à une teneur allant de 15 à 20 mg en nicotinamide.

Avantageusement la teneur en vitamine B3 dans le complément alimentaire correspond à la teneur maximale autorisée par la règlementation en vigueur.

La vitamine B5 correspond à l'acide pantothénique. Elle peut être présente dans le complément alimentaire sous la forme acide pantothénique ou sous la forme d'un sel de cet acide.

A titre de sels, on entend tout sel qui présente une innocuité adéquate, en particulier le pantothénate de calcium.

De préférence, le pantothénate de calcium est utilisé dans le complément alimentaire selon l'invention.

Le complément alimentaire selon la présente invention permet une administration journalière de vitamine B5 allant de 0,00001 g à 1g, de préférence de 0,0001 g à 0,5 g et de manière préférée de 0,0005 g à 0,1 g.

Avantageusement le complément alimentaire est formulé de façon à permettre une administration journalière en vitamine B5 correspondant à une teneur allant de 10 à 15 mg en pantothénate de calcium.

Avantageusement la teneur en vitamine B5 dans le complément alimentaire correspond à la teneur maximale autorisée par la règlementation en vigueur.

La vitamine B6 peut être présente dans le complément alimentaire sous la forme d'un ou plusieurs composés tels que la pyridoxine, l'acide pyridoxique, les esters de pyridoxine tel que le tripalmitate de pyridoxine, les amines de pyridoxine telle que la pyridoxamine, ainsi que leurs sels et dérivés.

A titre de sels, on entend tout sel qui présente une innocuité adéquate, en particulier le chlorhydrate de pyridoxine.

A titre de dérivés on peut citer les composés choisis dans le groupe formé par le pyridoxal, le phosphate de pyridoxal.

De préférence, le chlorhydrate de pyridoxine est utilisé dans le complément alimentaire selon l'invention.

Le complément alimentaire selon la présente invention permet une administration journalière de vitamine B6 allant de 0,00001 g à 1g, de préférence de 0,0001 g à 0,5 g et de manière préférée de 0,0005 g à 0,1 g.

Avantageusement le complément alimentaire est formulé de façon à permettre une administration journalière en vitamine B6 correspondant à une teneur allant de 0,1 à 0,6 mg en chlorhydrate de pyridoxine.

Avantageusement la teneur en vitamine B6 dans le complément alimentaire correspond à la teneur maximale autorisée par la règlementation en vigueur.

Le complément alimentaire selon la présente invention permet une administration journalière de vitamine B8 allant de 0,00001 g à 1g, de préférence de 0,0001 g à 0,5 g et de manière préférée de 0,0005 g à 0,1g.

Avantageusement le complément alimentaire est formulé de façon à permettre une administration journalière en vitamine B8 allant de 0,4 à 0,6 mg.

Avantageusement la teneur en vitamine B8 dans le complément alimentaire correspond à la teneur maximale autorisée par la règlementation en vigueur.

Bien entendu, les sels utilisés dans la formulation du complément alimentaire sont choisis pour leur innocuité. On peut notamment citer le sulfate de zinc et le sulfate de cuivre ainsi que leurs formes chélatées.

Avantageusement le complément alimentaire est formulé de façon à permettre une administration journalière en zinc allant de 8 à 12 mg.

Avantageusement la teneur en zinc dans le complément alimentaire correspond à la teneur maximale autorisée par la règlementation en vigueur.

Avantageusement le complément alimentaire est formulé de façon à permettre une administration journalière en cuivre allant de 1 à 2 mg.

Avantageusement la teneur en cuivre dans le complément alimentaire correspond à la teneur maximale autorisée par la règlementation en vigueur.

Avantageusement, le complément alimentaire comprend, en plus de la L-cystine contenue dans l'hydrolysat, de la L-cystine additionnelle, de préférence la quantité en poids de la L-cystine additionnelle est égale à la quantité en poids de l'hydrolysat.

De manière préférée le complément alimentaire comprend de la L-cystine additionnelle en une teneur allant de 0,001 à 2 g, de préférence de 0,5 à 1,5 g et de manière préférée environ 0,8 à 1,2 g.

Plus particulièrement, le complément alimentaire selon l'invention comprend :

un hydrolysat de kératine selon l'invention ou obtenu suivant le procédé de préparation selon l'invention en une teneur allant de 0,001 à 2 g, de préférence de 0,5 à 1,5 g et de manière préférée environ 0,8 à 1,2 g ;

de la vitamine B3 en une teneur correspondant à 1 à 3 % en poids de nicotinamide par rapport au poids de l'hydrolysat;

de la vitamine B5 en une teneur correspondant à 1 à 2 % en poids de pantothénate de calcium par rapport au poids de l'hydrolysat;

de la vitamine B6 en une teneur correspondant à 0,1 à 0,5 % en poids de chlorhydrate de pyridoxine par rapport au poids de l'hydrolysat;

de la vitamine B8 en une teneur allant de 0,01 à 0,1 % par rapport au poids de l'hydrolysat;

du zinc ou l'un de ses sels en une teneur allant de 0,5 à 2% de zinc par rapport au poids de l'hydrolysat;

du cuivre ou l'un de ses sels en une teneur allant de 0,05 à 0,5% de cuivre par rapport au poids de l'hydrolysat,

et éventuellement, de la L-cystine additionnelle en une quantité en poids égale à la quantité en poids de l'hydrolysat.

Le complément alimentaire conforme à l'invention comprend en outre un véhicule physiologiquement acceptable, conforme à une utilisation par voie orale.

Le complément alimentaire conforme à l'invention peut être formulé avec les excipients usuellement utilisés dans les compositions destinées à la voie orale, notamment des agents humectants, des épaississants, des agents de textures, des agents de saveur, des agents d'enrobage, des conservateurs, des antioxydants, des colorants, des extraits de plante comme par exemple un extrait de prèle.

Bien entendu, l'homme du métier veillera à choisir ces excipients de manière à ne pas altérer les propriétés du complément alimentaire.

Le complément alimentaire conforme à l'invention peut être formulé selon une des présentations suivantes:

une gélule, une dragée, un comprimé, une capsule molle ou dure, ou encore une suspension, une solution, un gel.

La formulation du complément alimentaire conforme à l'invention met en œuvre des procédés classiques qui font partie des compétences générale de l'homme du métier.

**Procédé de traitement cosmétique oral**

La présente invention concerne encore un procédé cosmétique pour améliorer la qualité et/ou l'aspect des ongles et/ou des cheveux notamment pour améliorer la qualité des cheveux comprenant l'administration par voie orale à un individu d'un hydrolysat selon la présente invention ou d'un complément alimentaire selon la présente invention tels que définis ci-dessus.

Ainsi le procédé selon la présente invention est un procédé cosmétique dans la mesure où il permet d'améliorer l'état et le caractère esthétique des ongles et/ou des cheveux. L'hydrolysat selon la présente invention et le complément alimentaire selon la présente invention peuvent être utilisés plusieurs mois sans prescription médicale ce qui les place clairement hors du domaine thérapeutique.

Avantageusement, le procédé selon la présente invention permet d'administrer à un individu les doses journalières suivantes :

un hydrolysat de kératine selon l'invention en une teneur allant de 0,001 à 2 g, de préférence de 0,5 à 1,5 g et de manière préférée environ 0,8 à 1,2 g ;

de la vitamine B3 en une teneur correspondant à une teneur allant de 0,015 à 0,020 g en nicotinamide ;

de la vitamine B5 en une teneur correspondant à une teneur allant de 0,010 à 0,015 g en pantothénate de calcium ;

de la vitamine B6 en une teneur correspondant à une teneur allant de 0,0004 à 0,0006 g en chlorhydrate de pyridoxine ;

de la vitamine B8 en une teneur allant de 0,0004 à 0,0006 g ;

du zinc ou l'un de ses sels en une teneur en zinc allant de 0,008 à 0,0012 g ;

du cuivre ou l'un de ses sels en une teneur en cuivre allant de 0,001 à 0,002 g ;

éventuellement de la L-cystine additionnelle en une quantité en poids égale à la quantité en poids de l'hydrolysat.

Le procédé selon l'invention est généralement mis en œuvre par une administration journalière de l'hydrolysat selon la présente invention ou du complément alimentaire selon la présente invention. Cette administration peut être effectuée en une seule prise journalière, en deux prises journalières, en trois prises journalières voire quatre prises journalières, par exemple aux heures des repas.

Le procédé selon l'invention est généralement mis en œuvre sur une période variant d'une à plusieurs semaines voire plusieurs mois. Cette période de traitement pouvant être répétée plusieurs fois au cours d'une année.

Les exemples qui suivent visent à illustrer l'invention.

Exemples

**Exemple 1- Hydrolysats**

**Préparation de l'hydrolysat I**

On introduit 4500 kg de plumes de volaille dans un réacteur/hydrolyseur de 15000 litres.

Une première étape d'hydrolyse chimique est réalisée en ajoutant 18 000 litres d'acide chlorhydrique (24%), l'hydrolyse est effectuée à 72°C pendant 4, 5 heures.

Le produit obtenu est stocké pendant 48 heures à température ambiante (pause intermédiaire) Ensuite une deuxième hydrolyse chimique est effectuée en chauffant à 107°C pendant 6 heures sans ajout d'acide.

Le produit obtenu est laissé à refroidir.

Ensuite une étape d'extraction de la tyrosine est réalisée en remontant le pH à environ 7 au moyen d'hydroxyde de sodium.

La solution obtenue est séchée par atomisation.

On obtient 4200 kg d'hydrolysat sous forme sèche.

**Hydrolysat II**

A titre de comparatif a été utilisé l'hydrolysat II qui est un hydrolysat issu de laine de mouton dont la composition est présentée dans le tableau 2.

**Détermination de la composition des hydrolysats**

Les acides aminés sont dosés selon une méthode adaptée du règlement CE 152/2009.
Les acides aminés sont séparés par chromatographie (CLHP ou « HPLC » en langue anglaise) avec colonne échangeuse d'ions et dosés par réaction avec la ninhydrine et détection photométrique à 570 nm.

Tableau 2

| Acide aminé/ peptide | Teneur dans l'hydrolysat I (% poids) | Teneur dans l'hydrolysat II (% poids) | Acide aminé/ peptide | Teneur dans l'hydrolysat I (% poids) | Teneur dans l'hydrolysat II (% poids) |
|---|---|---|---|---|---|
| Asp | 7,6 | 7,8 | Ile | 4,6 | 3,8 |
| Thr | 5,1 | 6,5 | Leu | 7,7 | 8,9 |
| Ser | 12,4 | 9,3 | Tyr | 0,3 | 3,1 |
| Glu | 11,4 | 15,8 | Phe | 2,5 | 3,2 |
| Gly | 8,6 | 4,8 | Lys | 2,0 | 2,7 |
| Ala | 5,0 | 4,5 | His | 0,7 | 0,8 |
| Val | 8,2 | 6,1 | Arg | 6,4 | 9,2 |
| Met | 0,2 | 0,5 | Pro | 11,8 | 5,9 |
| L-Cystine | 5,5 | 7,1 | **total** | **100** | **100** |

L'hydrolysat I comprend 6,6 % en poids de matières minérales et l'hydrolysat II comprend 29,6 % en poids de matière minérale.
L'hydrolysat I comprend 83,3 % en poids d'acides aminés libres, l'hydrolysat II comprend moins de 2 % en poids d'acides aminés libres par rapport au poids total de l'hydrolysat.

**Exemple 2-Digestibilité**

Dans le tableau 3, sont présentés les coefficients de digestibilité vraie de chacun des acides aminés des hydrolysats I et II, exprimés en pourcentage, ainsi que la valeur moyenne de digestibilité vraie du total des acides aminés.
Ces valeurs ont été obtenue selon le protocole suivant sur coqs caecectomisés, qui est un modèle de référence pour la mesure de la biodisponibilité des acides aminés dans le règne animal.

Protocole expérimental

Les mesures de digestibilité sont effectuées sur les coqs adultes caecectomisés logés dans des cages individuelles et nourris hors période d'essai avec un régime alimentaire standard.
Pour chaque hydrolysat, 2 répétitions de 4 coqs caecectomisés sont utilisées.
Tous les animaux sont mis à jeun pendant 24h avant d'ingérer un repas unique de 80 g composé de 24 g d'échantillon (hydrolysat I ou II) mélangé à 56 g de sucre.
Toutes les fèces (= matières fécales), y compris les pertes endogènes, sont collectées pendant les 48 h suivantes en deux périodes de 24h pour éviter leur fermentation et détérioration éventuelle.
Ces fèces, exclus de toutes contaminations telles des plumes par exemple, sont soigneusement prélevées avant d'être congelées (-80°C).
Les fèces sont ensuite séchées au four, regroupées et mélangées en 2 pools correspondant aux 2 répétitions de 4 animaux utilisés pour chacun des 2 hydrolysats.

Pour chaque hydrolysat, les 2 pools sont analysés.

Les analyses nutritionnelles (matière sèche, protéines brutes (méthode Dumas) et acides aminés) sont effectuées sur les hydrolysats, les fèces de coqs ainsi que sur les pertes endogènes.

Ces données sont utilisées pour calculer la digestibilité vraie des protéines et des acides aminés.

Pour la digestibilité des protéines, en raison de la contamination des excréments d'oiseaux par l'azote urique, l'azote protéique est mesuré dans les fèces (méthode Terpstra).

La digestibilité vraie des nutriments mesurée en pourcentage est donc calculée selon une méthode quantitative par la différence entre la quantité d' hydrolysat ingéré et la quantité de fèces excrétée, corrigée des pertes endogènes selon les formules suivantes :

$$\text{Digestibilité vraie protéines \%} = \frac{\text{Protéines ingérées hydrolysat - (protéines excrétées fèces – protéines excrétées endogènes)}}{\text{Protéines ingérées hydrolysat}} \times 100$$

$$\text{Digestibilité vraie A. aminé \%} = \frac{\text{Acide aminé ingéré hydrolysat - (Acide aminé excrété fèces – Acide aminé excrété endogène)}}{\text{Acide aminé ingéré hydrolysat}} \times 100$$

Les valeurs de digestibilité des hydrolysats I et II figurent dans le tableau 3 ci-dessous :

Tableau 3

| Acide aminé /peptide (AA) | Coefficient de digestibilité vraie des AA de l'hydrolysat I (%) | Coefficient de digestibilité vraie des AA de l'hydrolysat II (%) | Acide aminé /peptide (AA) | Coefficient de digestibilité vraie des AA de l'hydrolysat I (%) | Coefficient de digestibilité vraie des AA de l'hydrolysat II (%) |
|---|---|---|---|---|---|
| Asp | 97,55 | 73,88 | Ile | 98,28 | 78,61 |
| Thr | 95,70 | 72,64 | Leu | 98,64 | 84,24 |
| Ser | 98,91 | 75,72 | Tyr | 99,19 | 82,59 |
| Glu | 94,27 | 76,03 | Phe | 99,17 | 81,70 |
| Gly | 91,69 | 52,52 | Lys | 97,91 | 75,44 |
| Ala | 97,46 | 77,24 | His | 93,40 | 70,21 |
| Val | 98,04 | 76,85 | Arg | 96,25 | 78,74 |
| Met | 96,05 | 76,35 | Pro | 98,66 | 66,85 |
| L-Cystine | 84,39 | 69,26 | **Total** | **96.21** | **74.64** |

La digestibilité vraie des acides aminés de l'hydrolysat I selon l'invention est très élevée puisqu'elle est très proche du maximum possible (100%) et elle est très supérieure à celle des acides aminés l'hydrolysat comparatif II.

L'hydrolysat selon l'invention est par conséquent beaucoup mieux assimilable par l'organisme que l'hydrolysat comparatif, comme le montre sa digestibilité vraie moyenne des acides aminés qui est supérieure de 21,57 points à celle de l'hydrolysat comparatif.

**Exemple 3-Complément alimentaire**

**Préparation du complément alimentaire**

Tableau 4 - Complément alimentaire sous forme de gélule

| Les ingrédients suivant sont pesés, mélangés à température ambiante et mis en gélules. Les quantités sont indiquées en mg | | | |
|---|---|---|---|
| Ingrédients | Formule A | Formule B | Formule C |
| | Composition selon l'invention | Composition selon l'invention | (placebo) |
| Maltodextrine | 250 | 0 | 500 |
| Hydrolysat de kératine I | 250 | 250 | 0 |
| Hydrolysat de kératine II | 0 | 0 | 0 |
| L-Cystine | 0 | 250 | 0 |
| Stearate de magnesium | 14 | 14 | 14 |
| Sulfate de zinc (22% Zn) | 11,36 | 11,36 | 0 |
| Silice | 5 | 5 | 5 |
| Vitamine B3 (nicotinamide) m.a. 99% | 4,5 | 4,5 | 0 |
| Vitamine B5 (D pantothenate de calcium) m.a. 92% | 3,72 | 3,72 | 0 |
| Extrait sec de partie aérienne de prêle | 2,5 | 2,5 | 0 |
| Sulfate de cuivre (25% Cu) | 1,48 | 1,48 | 0 |
| Vitamine B6 (chlorhydrate de pyridoxine) m.a. 83% | 0,63 | 0,63 | 0 |
| Vitamine B8 (biotine) m.a. 99,75% | 0,15 | 0,15 | 0 |

**Etude**

Le complément alimentaire a été administré à raison de 4 gélules par jour (2 gélules le matin et 2 gélules le soir) pendant 90 jours.

L'étude a été réalisée sur 60 sujets, en double aveugle, avec répartition aléatoire et en présence d'un placebo (20 sujets ont reçu la Formule A, 20 sujets ont reçu la Formule B et 20 sujets ont reçu la Formule C placebo)

Les sujets retenus répondaient aux critères suivants :

- sujets en bonne santé, âgés de 30 à 60 ans,

- cheveux de couleur naturelle foncée : bruns ou noirs, dans chaque groupe 20% des sujets avaient antérieurement effectués une décoloration des cheveux,

- sujet ayant tendance à perdre des cheveux et présentant des ongles fragiles.

Cheveux

Sur les cheveux, la brillance, la présence d'effluvium télogène, la densité et le volume ont été évalués.

La brillance a été mesurée en utilisant un spectrophotomètre/colorimètre CM 700D (Konica-Minolta). Le paramètre mesuré était la valeur de brillance (« gloss value » en langue anglaise) à 8°.

La présence d'effluvium télogène est mesurée au moyen d'un test de résistance à la traction (« pull test» en langue anglaise). La résistance à la traction a été utilisée pour déterminer la qualité de l'ancrage des cheveux à la papille pilaire. Une traction douce est exercée sur des mèches de cheveux (environ 60 cheveux) en trois zones différentes de la chevelure : zone frontale, zone occipitale et zone temporale et le nombre de cheveux arrachés est compté.

Normalement moins de trois cheveux en phase télogène doivent être arrachés à chaque traction.

Si au moins trois cheveux en phase télogène sont arrachés à chaque traction ou si plus de dix cheveux au total sont arrachés, le test de résistance à la traction est considéré positif et révèle la présence d'effluvium télogène.

La densité mesurée en nombre de cheveux par $cm^2$ est obtenue avec un phototrichogramme, établi selon les recommandations de la méthode déposée de référence Trichoscan®.

Le volume de la chevelure est déterminé par un dermatologue par comparaison de photographies digitales prises à t=0, à 45 jours et à 90 jours. L'appareil photo utilisé est un NIKON D300/600 équipé d'un macro-objectif, d'un flash indépendant et de filtres polarisés (croisé ou parallèle). Pour chaque sujet, 4 photos sont prises : deux dans la zone du vertex (une avec un filtre polarisé croisé et une avec un filtre polarisé parallèle) et deux dans la zone frontale (une avec un filtre polarisé croisé et une avec un filtre polarisé parallèle).

A chaque fois des notes sont données puis le nombre de sujets présentant une augmentation de la chevelure est déterminé.

Ongles

Sur les ongles, la brillance et l'aspect/qualité ont été évalués.

La brillance a été mesurée en utilisant un spectrophotomètre/colorimètre CM 700D (Konica-Minolta). Le paramètre mesuré était la valeur de brillance « gloss value » en langue anglaise, à 8°.

La qualité/aspect des ongles est déterminée notation par un dermatologue.

Tableau 5

| Paramètre | Formule A Testée à 45 jours | Formule A Testée à 90 jours | Formule B Testée à 45 jours | Formule B Testée à 90 jours |
|---|---|---|---|---|
| **cheveux** | | | | |
| % anagènes | 82,3* ± 0,7 (+3,6%) | 87,7*±0,6 (+9,1%) | 82,7* ± 0,7 (+4,3%) | 88,2* ± 0,5 (+9,7%) |
| % télogènes | 17,8*± 0,7 (- 3,6%) | 12,3*± 0,6 (- 9,1%) | 17,3*± 0,7 (- 4,3%) | 11,8*± 0,5 (- 9,7%) |
| Test de traction (pull test) /t0 | 8,9*± 0,4 (- 26,5%) | 8,1*± 0,4 (- 32,7%) | 9,6*± 0,5 (- 24,3%) | 8,3*± 0,3 (- 34,0%) |
| Brillance/t0 | 3,98*± 0,45 (+23,8%) | 4,99*± 0,53 (+57,0%) | 4,08*± 0,38) (+31,4%) | 4,85*± 0,43 (+58,9%) |
| Volume** | 40,0%* | 65,0%* | 40,0%* | 75,0%* |
| Densité | 194,7±6,7 (+2,1%) | 205,9*±6,4 (+13,3%) | 198,9±4,8 (+3,4%) | 211,3*±4,9 (+15,8%) |
| **Ongles** | | | | |

| Brillance /t0 | 9,54*± 0,93 (+33,1%) | 9,57± 0,86 (+35,7%) | 9,02*± 0,92 (+28,5%) | 10,74*± 0,99 (+58,4%) |
|---|---|---|---|---|
| Amélioration de l'aspect notée par un dermatologue | 30,0% | 60,0%* | 35,0% | 70,0%* |

| Paramètre | Formule C Testée à 45 jours | Formule C Testée à 90 jours |
|---|---|---|
| cheveux | | |
| % anagènes | 78,6 ± 0,7 (-0,4%) | 82,2 ± 0,7 (+3,2%) |
| % télogènes | 21,4± 0,7 (+0,4%) | 17,9± 0,7 (-3,2%) |
| Test de traction (pull test) /t0 | 12,4± 0,5 (+1,0%) | 11,0± 0,5(- 10,6%) |
| Brillance/t0 | 3,28± 0,36 (-1,9%) | 3,53±0,39 (+6,4%) |
| Volume** | 5,0% | 10,0% |
| Densité | 194,0 ± 7,1 (+0,2%) | 195,0 ± 7,2 (+1,8%) |
| Ongles | | |
| Brillance /t0 | 7,76± 0,59 (+6,7%) | 8,45±0,69 (+17,2%) |
| Amélioration de l'aspect notée par un dermatologue | 15,0% | 20,0% |

* : Significativement différent de la formule placebo

( ) : entre parenthèses, pourcentage d'augmentation par rapport au début de l'essai

** : % de sujets présentant une augmentation du volume de leur chevelure.

Tableau 6

| | | | | | | |
|---|---|---|---|---|---|---|
| La croissance des ongles est mesurée à partir de photographies digitales en utilisant une technique d'analyse d'image morphométrique. Les ongles sont coupés et la croissance est mesurée entre J-7 et J+7 (mesure t=0) puis entre J38 et J52 (mesure t=45) puis entre J83 et J102 (mesure t=90). | | | | | | |
| Croissance des ongles | Form. A Testée à t=0 | Form. A Testée à 45 jours | Form. A Testée à 90 jours | Form. B Testée à t=0 | Form. B Testée à 45 jours | Form. B Testée à 90 jours |
| | $1,24 \pm 0.07$ | $1,31 \pm 0.07$ | $1,38* \pm 0.08$ | $1,21 \pm 0.05$ | $1,30* \pm 0.06$ | $1,43* \pm 0.07$ |
| | Form. C Testée à t=0 | Form. C Testée à 45 jours | Form. C Testée à 90 jours | | | |
| | $1,26 \pm 0.06$ | $1,28 \pm 0.05$ | $1,28 \pm 0.06$ | | | |
| * Significativement différent de la formule C (placebo) | | | | | | |

Résultats

Sur les cheveux

La formule A, conforme à l'invention, permet une amélioration de l'ancrage des cheveux, de la brillance des cheveux, de la densité et du volume de la chevelure.

L'association de l'hydrolysat selon l'invention avec de la L-Cystine additionnelle, formule B conforme à l'invention, permet d'améliorer encore les résultats obtenus.

Sur les ongles

La formule A, conforme à l'invention, permet une augmentation de la brillance, de la qualité et de la croissance des ongles.

Ces résultats montrent que l'administration de la formule A comprenant un hydrolysat de kératine, des vitamines, du cuivre et du zinc permet l'amélioration de l'aspect des cheveux et des ongles par rapport à la formule placebo.

De plus l'association de l'hydrolysat selon l'invention avec la L-cystine additionnelle permet d'améliorer encore les résultats.

**Revendications**

1.   Hydrolysat de kératine **caractérisé en ce que** :

ledit hydrolysat comprend au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton,
ledit hydrolysat comprend de la L-cystine en une teneur allant de 4 à 6% en poids par rapport au poids total de l'hydrolysat,
ledit hydrolysat comprend de la cystéine en une teneur inférieure ou égale à 0,1% en poids par rapport au poids total de l'hydrolysat,
ledit hydrolysat comprend de la tyrosine en une teneur inférieure ou égale à 0,6% en poids par rapport au poids total de l'hydrolysat.

2.   Hydrolysat de kératine selon la revendication 1 **caractérisé en ce qu'**il présente la composition suivante en acides aminés totaux : une teneur en glycine allant de 7 à 10 % en poids ; une teneur en alanine allant de 4 à 6 % en poids ; une teneur en valine allant de 6 à 10 % en poids ; une teneur en proline allant de 9 à 14 % en poids.

**3.** Hydrolysat de kératine selon la revendication 1 ou 2 **caractérisé en ce qu'**il comprend la composition suivante en acides aminés totaux : une teneur en acide aspartique allant de 6 à 9 % en poids, de préférence 7,6 % en poids ; une teneur en thréonine allant de 4 à 6 % en poids, de préférence de 5,1 % en poids ; une teneur en sérine allant de 10 à 15 % en poids, de préférence de 12,4 % en poids ; une teneur en acide glutamique allant de 9 à 14 % en poids, de préférence de 11,4 % en poids ; une teneur en glycine allant de 7 à 10 % en poids, de préférence de 8,6 % en poids ; une teneur en alanine allant de 4 à 6 % en poids, de préférence de 5,0 % en poids ; une teneur en valine allant de 6 à 10 % en poids, de préférence 8,2 % en poids ; une teneur en méthionine allant de 0,1 à 0,4 % en poids, de préférence de 0,2 % en poids ; une teneur en isoleucine allant de 4 à 6 % en poids, de préférence de 4,6 % en poids ; une teneur en leucine allant de 6 à 9 % en poids, de préférence 7,7 % en poids ; une teneur en tyrosine allant de 0,1 à 0,5 % en poids, de préférence 0,3 % en poids ; une teneur en phénylalanine allant de 2 à 3 % en poids, de préférence 2,5 % en poids ; une teneur en lysine allant de 1 à 3 % en poids, de préférence 2,0 % en poids, une teneur en histidine allant de 0,4 à 1 % en poids, de préférence 0,7 % en poids ; une teneur en arginine allant de 5 à 8 % en poids, de préférence 6,4 % en poids ; une teneur en proline allant de 9 à 14 % en poids, de préférence 11,8 % en poids, une teneur en tryptophane inférieure à 0.1 % ,de préférence 0 % en poids et une teneur en L-Cystine allant de 4 à 6 % en poids, de préférence 5,5 % en poids.

**4.** Hydrolysat selon l'une quelconque des revendications 1 à 3 **caractérisé en ce qu'**il présente une digestibilité vraie des acides aminés d'au moins 90%.

**5.** Procédé de préparation de l'hydrolysat de kératine selon l'une quelconque des revendications 1 à 4 à partir d'une matière première kératinique de volaille **caractérisé en ce qu'**il comprend au moins les étapes suivantes, dans cet ordre :

- soumettre la matière première à au moins une hydrolyse chimique au moyen d'un acide dans des conditions aptes à obtenir un hydrolysat comprenant au moins 88% en poids d'acides aminés libres par rapport au poids total des acides aminés de l'hydrolysat, le reste des acides aminés de l'hydrolysat étant sous la forme de peptides présentant une masse moléculaire inférieure ou égale à 800 Dalton,
- extraire la tyrosine dudit hydrolysat,
- éventuellement sécher.

**6.** Procédé selon la revendication précédente **caractérisé en ce que** l'hydrolyse chimique est effectuée en deux étapes :

- une première hydrolyse chimique réalisée à une température allant de 60 à 80°C pendant une période allant de 4 à 5 heures puis
- une deuxième hydrolyse chimique réalisée à une température allant de 100 à 115°C pendant une période allant de 5 à 7 heures,

les deux hydrolyses pouvant être réalisées sans étape de pause intermédiaire ou en effectuant une étape de pause intermédiaire comprise entre 1 heure et 7 jours.

**7.** Utilisation cosmétique, par voie orale, de l'hydrolysat de kératine selon l'une quelconque des revendications 1 à 4 ou obtenu selon le procédé suivant l'une quelconque des revendications 5 ou 6 comme agent pour améliorer la qualité et/ou l'aspect des ongles et/ou des cheveux, notamment pour améliorer l'ancrage, la brillance et/ou le volume et/ou la densité des cheveux.

**8.** Complément alimentaire comprenant un hydrolysat de kératine selon l'une quelconque des revendications 1 à 4 ou obtenu selon le procédé suivant l'une quelconque des revendications 5 ou 6, ledit complément alimentaire comprenant de 40 à 60% en poids d'hydrolysat de kératine par rapport au poids total dudit complément alimentaire.

**9.** Complément alimentaire selon la revendication 8 **caractérisé en ce que**, à l'exception de la L-cystine, ledit complément alimentaire ne comprend pas d'autres acides aminés libres que ceux contenus dans l'hydrolysat.

**10.** Complément alimentaire selon l'une quelconque des revendications 8 ou 9 tel qu'il comprend également au moins un composant choisi dans le groupe formé par le zinc ou l'un de ses sels, le cuivre ou l'un de ses sels, les vitamines B, notamment B3, B5, B6 et/ou B8, et les mélanges de ces composants.

**11.** Complément alimentaire selon l'une quelconque des revendications 8 à 10 tel qu'il comprend :

un hydrolysat de kératine selon l'une quelconque des revendications 1 à 4 ou obtenu selon le procédé suivant l'une quelconque des revendications 5 ou 6 en une teneur allant de 0,001 à 2 g, de préférence de 0,5 à 1,5 g et de manière préférée environ 0,8 à 1,2 g ;

de la vitamine B3 en une teneur correspondant à 1 à 3 % en poids de nicotinamide par rapport au poids de l'hydrolysat;

de la vitamine B5 en une teneur correspondant à 1 à 2 % en poids de pantothénate de calcium par rapport au poids de l'hydrolysat;

de la vitamine B6 en une teneur correspondant à 0,1 à 0,5 % en poids de chlorhydrate de pyridoxine par rapport au poids de l'hydrolysat;

de la vitamine B8 en une teneur allant de 0,01 à 0,1 % par rapport au poids de l'hydrolysat;

du zinc ou l'un de ses sels en une teneur allant de 0,5 à 2% de zinc par rapport au poids de l'hydrolysat;

du cuivre ou l'un de ses sels en une teneur allant de 0,05 à 0,5% de cuivre par rapport au poids de l'hydrolysat.

**12.** Complément alimentaire selon l'une quelconque des revendications 8 à 11 **caractérisé en ce qu'**il comprend, en plus de la L-cystine contenue dans l'hydrolysat, de la L-cystine additionnelle, de préférence la quantité en poids de la L-cystine additionnelle est égale à la quantité en poids de l'hydrolysat.

**13.** Procédé de traitement cosmétique pour améliorer la qualité et/ou l'aspect des ongles et/ou des cheveux, notamment pour améliorer la qualité des cheveux, comprenant l'administration, par voie orale, à un individu, d'un hydrolysat selon l'une quelconque des revendications 1 à 4 ou obtenu selon le procédé suivant l'une quelconque des revendications 5 ou 6 ou d'un complément alimentaire selon l'une quelconque des revendications 8 à 12.

**14.** Procédé selon la revendication précédente dans lequel on administre à un individu les doses journalières suivantes

un hydrolysat de kératine en une teneur allant de 0,001 à 2 g, de préférence de 0,5 à 1,5 g et de manière préférée environ 0,8 à 1,2 g ;

de la vitamine B3 en une teneur correspondant à une teneur allant de 0,015 à 0,020 g en nicotinamide ;

de la vitamine B5 en une teneur correspondant à une teneur allant de 0.010 à 0,015 g en pantothénate de calcium ;

de la vitamine B6 en une teneur correspondant à une teneur allant de 0,0004 à 0,0006 g en chlorhydrate de pyridoxine ;

de la vitamine B8 en une teneur allant de 0,0004 à 0,0006 g ;

du zinc ou l'un de ses sels en une teneur en zinc allant de 0,008 à 0,0012 g ;

du cuivre ou l'un de ses sels en une teneur en cuivre allant de 0,001 à 0,002 g.

**15.** Procédé selon la revendication 14 dans lequel on administre en outre de la L-cystine additionnelle en une quantité en poids égale à la quantité en poids de l'hydrolysat.

**Patentansprüche**

**1.** Keratinhydrolysat, **dadurch gekennzeichnet, dass**:

das Hydrolysat mindestens 88 Gew.-% freie Aminosäuren bezogen auf das Gesamtgewicht der Aminosäuren des Hydrolysats umfasst, wobei der Rest der Aminosäuren des Hydrolysats in Form von Peptiden mit einem Molekulargewicht kleiner/gleich 800 Dalton auftritt,

das Hydrolysat einen L-Cystin-Anteil am Gesamtgewicht des Hydrolysats von 4 bis 6 Gew.-% umfasst,

das Hydrolysat einen Cystein-Anteil am Gesamtgewicht des Hydrolysats kleiner/gleich 0,1 Gew.-% umfasst,

das Hydrolysat einem Tyrosin-Anteil am Gesamtgewicht des Hydrolysats kleiner/gleich 0,6 Gew.-% umfasst.

**2.** Keratinhydrolysat nach Anspruch 1, **dadurch gekennzeichnet, dass** es die folgende Gesamtzusammensetzung an Aminosäuren aufweist: einen Glycin-Anteil von 7 bis 10 Gew.-%; einen Alanin-Anteil von 4 bis 6 Gew.-%; einen Valin-Anteil von 6 bis 10 Gew.- %; einen Prolin-Anteil von 9 bis 14 Gew.-%.

**3.** Keratinhydrolysat nach Anspruch 1 oder 2, **dadurch gekennzeichnet, dass** es die folgende Gesamtzusammensetzung an Aminosäuren umfasst: einen Asparaginsäure-Anteil von 6 bis 9 Gew.-%, vorzugsweise 7,6 Gew.-%; einen Threonin-Anteil von 4 bis 6 Gew.-%, vorzugsweise 5,1 Gew.-%; einen Serin-Anteil von 10 bis 15 Gew.-%, vorzugsweise 12,4 Gew.-%; einen Glutaminsäure-Anteil von 9 bis 14 Gew.-%, vorzugsweise 11,4 Gew.-%; einen Glycingehalt von 7 bis 10 Gew.-%, vorzugsweise 8,6 Gew.-%; einen Alanin-Anteil von 4 bis 6 Gew.-%, vorzugsweise 5,0 Gew.-%; einen Valin-Anteil von 6 bis 10 Gew.-%, vorzugsweise 8,2 Gew.-%; einen Methionin-Anteil von 0,1 bis 0,4 Gew.-%, vorzugsweise 0,2 Gew.-%; einen Isoleucin-Anteil von 4 bis 6 Gew.-%, vorzugsweise 4,6 Gew.-%; einen Leucin-Anteil von 6 bis 9 Gew.-%, vorzugsweise 7,7 Gew.-%; einen Tyrosin-Anteil von 0,1 bis 0,5 Gew.-%, vorzugsweise 0,3 Gew.-%; einen Phenylalanin-Anteil von 2 bis 3 Gew.-%, vorzugsweise 2,5 Gew.-%; einen Lysin-Anteil von 1 bis 3 Gew.-%, vorzugsweise 2,0 Gew.- %, einen Histidin-Anteil von 0,4 bis 1 Gew.-%, vorzugsweise 0,7 Gew.-%; einen Arginin-Anteil von 5 bis 8 Gew.-%, vorzugsweise 6,4 Gew.-%; einen Prolin-Anteil von 9 bis 14 Gew.-%, vorzugsweise 11,8 Gew.-%, einen Tryptophan-Anteil kleiner 0,1 Gew.-%, vorzugsweise 0 Gew.-% und einen L-Cystin-Anteil von 4 bis 6 Gew.-%, vorzugsweise 5,5 Gew.-%.

4. Hydrolysat nach einem der Ansprüche 1 bis 3, **dadurch gekennzeichnet, dass** es eine tatsächliche Aminosäureverdaulichkeit von mindestens 90 % aufweist.

5. Verfahren zur Herstellung des Keratinhydrolysats nach einem der Ansprüche 1 bis 4 aus keratinischen Rohmaterial von Geflügel, **dadurch gekennzeichnet, dass** es mindestens die folgenden Schritte in der aufgeführten Reihenfolge umfasst:

   - das Rohmaterial mittels einer Säure mindestens einer chemischen Hydrolyse unterziehen, deren Bedingungen geeignet sind, ein Hydrolysat zu erhalten, das mindestens 88 Gew.-% freie Aminosäuren des Gesamtgewichts der Aminosäuren des Hydrolysats umfasst, wobei der Rest der Aminosäuren des Hydrolysats in Form von Peptiden mit einem Molekulargewicht kleiner/gleich 800 Dalton auftritt,
   - das Tyrosin aus dem Hydrolysat extrahieren,
   - eventuell trocken.

6. Verfahren nach dem vorhergehenden Anspruch, **dadurch gekennzeichnet, dass** die chemische Hydrolyse in zwei Schritten durchgeführt wird:

   - eine erste chemische Hydrolyse, die bei einer Temperatur von 60 bis 80 °C 4 bis 5 Stunden lang durchgeführt wird, dann
   - eine zweite chemische Hydrolyse, die bei einer Temperatur von 100 bis 115 °C 5 bis 7 Stunden durchgeführt wird,

   wobei die beiden Hydrolysen ohne Zwischenpause oder mit einer Zwischenpause von 1 Stunde bis 7 Tagen durchgeführt werden können.

7. Kosmetische, orale Verwendung des Keratinhydrolysats nach einem der Ansprüche 1 bis 4 oder eines Keratinhydrolysats, das gemäß dem Verfahren nach einem der Ansprüche 5 oder 6 erhalten wird als Mittel zur Verbesserung der Beschaffenheit und/oder des Aussehens der Nägel und/oder oder des Haars, insbesondere zur Verbesserung der Verankerung, des Glanzes und/oder des Volumens und/oder der Dichte des Haares.

8. Nahrungsergänzungsmittel, das ein Keratinhydrolysat nach einem der Ansprüche 1 bis 4 umfasst oder ein Keratinhydrolysat, das gemäß dem Verfahren nach einem der Ansprüche 5 oder 6 erhalten wird, wobei das Nahrungsergänzungsmittel 40 bis 60 Gew.-% Keratinhydrolysat bezogen auf das Gesamtgewicht des Nahrungsergänzungsmittels umfasst.

9. Nahrungsergänzungsmittel nach Anspruch 8, **dadurch gekennzeichnet, dass** das Nahrungsergänzungsmittel mit Ausnahme von L-Cystin keine anderen freien Aminosäuren als die in dem Hydrolysat enthaltenen umfasst.

10. Nahrungsergänzungsmittel nach einem der Ansprüche 8 oder 9, das darüber hinaus mindestens einen Bestandteil aus der Gruppe umfasst, die gebildet wird aus Zink oder eines seiner Salze, aus Kupfer oder eines seiner Salze, B-Vitaminen, insbesondere B3, B5, B6 und/oder B8, und den Mischungen dieser Bestandteile.

11. Nahrungsergänzungsmittel nach einem der Ansprüche 8 bis 10, das folgendes umfasst:

   ein Keratinhydrolysat nach einem der Ansprüche 1 bis 4 oder ein Keratinhydrolysat, das gemäß dem Verfahren nach einem der Ansprüche 5 oder 6 erhalten wird in einem Anteil von 0,001 bis 2 g, vorzugsweise 0,5 bis 1,5 g und im Idealfall etwa 0,8 bis 1,2 g;
   Vitamin B3 in einem Anteil, der einem Nicotinamid-Anteil von 1 bis 3 Gew.-% am Gewicht des Hydrolysats entspricht;
Vitamin B5 in einem Anteil, der einem Calciumpantothenat-Anteil von 1 bis 2 Gew.-% am Gewicht des Hydrolysats entspricht;
Vitamin B6 in einem Anteil, der einem Pyridoxinhydrochlorid-Anteil von 0,1 bis 0,5 Gew.-% am Gewicht des Hydrolysats entspricht;
Vitamin B8 in einem Anteil von 0,01 bis 0,1 Gew.-% am Gewicht des Hydrolysats;
Zink oder eines seiner Salze in einem Anteil, der einem Zink-Anteil von 0,5 bis 2 % am Gewicht des Hydrolysats entspricht;
Kupfer oder eines seiner Salze in einem KupferAnteil von 0,05 bis 0,5 % am Gewicht des Hydrolysats.

12. Nahrungsergänzungsmittel nach einem der Ansprüche 8 bis 11, **dadurch gekennzeichnet, dass** es neben dem im Hydrolysat enthaltenen L-Cystin auch zusätzliches L-Cystin enthält, wobei die Gewichtsmenge des zusätzlichen L-Cystins vorzugsweise gleich der Gewichtsmenge des Hydrolysats ist.

13. Kosmetisches Behandlungsverfahren zur Verbesserung der Beschaffenheit und/oder des Aussehens der Nägel und/oder des Haars, insbesondere aber zur Verbesserung der Beschaffenheit des Haars, das die orale Verabreichung eines Hydrolysats nach einem der Ansprüche 1 bis 4 oder eines Hydrolysats, das gemäß dem Verfahren nach einem der Ansprüche 5 oder 6 erhalten wird, oder die Verabreichung eines Nahrungsergänzungsmittels nach einem der Ansprüche 8 bis 12 an ein Individuum umfasst.

14. Verfahren nach dem vorhergehenden Anspruch, in dem man einem Individuum die folgenden Tagesdosen verabreicht:

Keratinhydrolysat in einem Anteil von 0,001 bis 2 g, vorzugsweise von 0,5 bis 1,5 g und im Idealfall etwa 0,8 bis 1,2 g;
Vitamin B3 in einem Anteil, der einem Nicotinamid-Anteil von 0,015 bis 0,020 g entspricht;
Vitamin B5 in einem Anteil, der einem Calciumpantothenat-Anteil von 0,010 bis 0,015 g entspricht;
Vitamin B6 in einem Anteil, der einem Pyridoxinhydrochlorid-Anteil von 0,0004 bis 0,0006 g entspricht;
Vitamin B8 in einem Anteil von 0,0004 bis 0,0006 g;
Zink oder eines seiner Salze in einem Zink-Anteil von 0,008 bis 0,0012 g;
Kupfer oder eines seiner Salze in einem KupferAnteil von 0,001 bis 0,002 g.

15. Verfahren nach Anspruch 14, in dem man des Weiteren zusätzliches L-Cystin in einer Gewichtsmenge gleich der Gewichtsmenge des Hydrolysats verabreicht.

**Claims**

1. A keratin hydrolysate **characterized in that**:

said hydrolysate comprises at least 88% by weight of free amino acids relative to the total weight of the amino acids of the hydrolysate, the remainder of the amino acids of the hydrolysate being in the form of peptides having a molecular mass less than or equal to 800 Dalton,
said hydrolysate comprises L-cystine in a content ranging from 4% to 6% by weight relative to the total weight of the hydrolysate,
said hydrolysate comprises cysteine in a content less than or equal to 0.1% by weight relative to the total weight of the hydrolysate,
said hydrolysate comprises tyrosine in a content less than or equal to 0.6% by weight relative to the total weight of the hydrolysate.

2. The keratin hydrolysate as claimed in claim 1, **characterized in that** it has the following composition of total amino acids: a glycine content ranging from 7% to 10% by weight; an alanine content ranging from 4% to 6% by weight; a valine content ranging from 6% to 10% by weight; a proline content ranging from 9% to 14% by weight.

3. The keratin hydrolysate as claimed in claim 1 or 2, **characterized in that** it comprises the following composition of total amino acids: an aspartic acid content ranging from 6% to 9% by weight, preferably 7.6% by weight; a threonine content ranging from 4% to 6% by weight, preferably 5.1% by weight; a serine content ranging from 10% to 15% by weight, preferably 12.4% by weight; a glutamic acid content ranging from 9% to 14% by weight, preferably 11.4% by weight; a glycine content ranging from 7% to 10% by weight, preferably 8.6% by weight; an alanine content ranging from 4% to 6% by weight, preferably 5.0% by weight; a valine content ranging from 6% to 10% by weight, preferably 8.2% by weight; a methionine content ranging from 0.1% to 0.4% by weight, preferably 0.2% by weight; an isoleucine content ranging from 4% to 6% by weight, preferably 4.6% by weight; a leucine content ranging from 6% to 9% by weight, preferably 7.7% by weight; a tyrosine content ranging from 0.1% to 0.5% by weight, preferably 0.3% by weight; a phenylalanine content ranging from 2% to 3% by weight, preferably 2.5% by weight; a lysine content ranging from 1% to 3% by weight, preferably 2.0% by weight, a histidine content ranging from 0.4% to 1% by weight, preferably 0.7% by weight; an arginine content ranging from 5% to 8% by weight, preferably 6.4% by weight; a proline content ranging from 9% to 14% by weight, preferably 11.8% by weight, a tryptophan content of less than 0.1%, preferably 0% by weight and an L-cystine content ranging from 4% to 6% by weight, preferably 5.5% by weight.

4. The hydrolysate as claimed in any one of claims 1 to 3, **characterized in that** it has a true digestibility of the amino acids of at least 90%.

5. A method for preparing the keratin hydrolysate as claimed in any one of claims 1 to 4 from a poultry keratin raw material, **characterized in that** it comprises at least the following steps, in this order:

- subjecting the raw material to at least one chemical hydrolysis by means of an acid under conditions suitable for obtaining a hydrolysate comprising at least 88% by weight of free amino acids relative to the total weight of the amino acids of the hydrolysate, the remainder of the amino acids of the hydrolysate being in the form of peptides having a molecular mass less than or equal to 800 Dalton,
- extracting the tyrosine from said hydrolysate,
- optionally drying.

6. The method as claimed in the preceding claim, **characterized in that** the chemical hydrolysis is carried out in two steps:

- a first chemical hydrolysis carried out at a temperature ranging from 60°C to 80°C for a period ranging from 4 to 5 hours, then
- a second chemical hydrolysis carried out at a temperature ranging from 100°C to 115°C for a period ranging from 5 to 7 hours,

it being possible for the two hydrolyses to be carried out without an intermediate waiting step or by staging an intermediate waiting step of between 1 hour and 7 days.

7. The cosmetic use, orally, of the keratin hydrolysate as claimed in any one of claims 1 to 4 or obtained according to the method as claimed in either one of claims 5 and 6, as an agent for improving the quality and/or the appearance of the nails and/or of the hair, notably for improving the anchoring, the gloss and/or the volume and/or the density of the hair.

8. A dietary supplement comprising a keratin hydrolysate as claimed in any one of claims 1 to 4 or obtained according to the method as claimed in either one of claims 5 and 6, said dietary supplement comprising from 40% to 60% by weight of keratin hydrolyzate relative to the total weight of said dietary supplement.

9. The dietary supplement as claimed in claim 8, **characterized in that**, with the exception of L-cystine, said dietary supplement comprises no free amino acids other than those contained in the hydrolyzate.

10. The dietary supplement as claimed in either one of claims 8 and 9, such that it also comprises at least one component selected from the group consisting of zinc or a salt thereof, copper or a salt thereof, B vitamins, notably vitamin B3, B5, B6 and/or B8, and the mixtures of these components.

11. The dietary supplement as claimed in any one of claims 8 to 10, such that it comprises:

a keratin hydrolysate as claimed in any one of claims 1 to 4 or obtained according to the method as claimed in either one of claims 5 and 6, in a content ranging from 0.001 to 2 g, preferably from 0.5 to 1.5 g and preferably around 0.8 to 1.2 g;
vitamin B3 in a content corresponding to 1% to 3% by weight of nicotinamide relative to the weight of the hydrolysate;

vitamin B5 in a content corresponding to 1% to 2% by weight of calcium pantothenate relative to the weight of the hydrolysate;

vitamin B6 in a content corresponding to 0.1% to 0.5% by weight of pyridoxine hydrochloride relative to the weight of the hydrolysate;

vitamin B8 in a content ranging from 0.01% to 0.1% relative to the weight of the hydrolysate;

zinc or a salt thereof in a content ranging from 0.5% to 2% of zinc relative to the weight of the hydrolysate;

copper or a salt thereof in a content ranging from 0.05% to 0.5% of copper relative to the weight of the hydrolysate.

12. The dietary supplement as claimed in any one of claims 8 to 11, **characterized in that** it comprises, besides the L-cystine contained in the hydrolysate, additional L-cystine, preferably the amount by weight of the additional L-cystine is equal to the amount by weight of the hydrolysate.

13. A cosmetic treatment method for improving the quality and/or the appearance of the nails and/or of the hair, notably for improving the quality of the hair, comprising the oral administration, to an individual, of a hydrolysate as claimed in any one of claims 1 to 4 or obtained according to the method as claimed in either one of claims 5 and 6, or of a dietary supplement as claimed in any one of claims 8 to 12.

14. The method as claimed in the preceding claim, wherein the following daily doses are administered to an individual:

a keratin hydrolyzate in a content ranging from 0.001 to 2 g, preferably from 0.5 to 1.5 g and preferably around 0.8 to 1.2 g;

vitamin B3 in a content corresponding to a content ranging from 0.015 to 0.020 g of nicotinamide;

vitamin B5 in a content corresponding to a content ranging from 0.010 to 0.015 g of calcium pantothenate;

vitamin B6 in a content corresponding to a content ranging from 0.0004 to 0.0006 g of pyridoxine hydrochloride;

vitamin B8 in a content ranging from 0.0004 to 0.0006 g;

zinc or a salt thereof in a zinc content ranging from 0.008 to 0.0012 g;

copper or a salt thereof in a copper content ranging from 0.001 to 0.002 g.

15. The method as claimed in claim 14, wherein additional L-cystine is furthermore administered in an amount by weight equal to the amount by weight of the hydrolysate.

**EP 3 675 811 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 200788402 A

**Littérature non-brevet citée dans la description**

- **COZANNET P. ; PRIMOT Y. ; GADY C. ; MÉTAYER J.P. ; LESSIRE M. ; SKIBA F. ; NOBLET J.** Standardised amino acid digestibility of wheat distillers' dried grains with solubles in force-fed cockerels. *British Poultry Science,* Février 2011, vol. 52 (1), 72-81